# EUROPEAN PATENT APPLICATION

(11) **EP 3 156 014 A1**
(43) Date of publication of application: **19.04.2017**
(21) Application number: 16020364.2
(22) Date of filing: 01.10.2016
(51) Int. Cl.: A61F 9/008

(54) **PATTERN SCANNING OPHTHALMIC ENDOLASER PROBE SYSTEM**

(30) Priority: 07.10.2015 US 201562238500 P; 30.09.2016 US 201615282491
(71) Applicant: Mehmet Melek, Istanbul 34457 (TR)
(72) Inventor: Mehmet Melek, Istanbul 34457 (TR)

(57) **Abstract**

A multiple-fiber pattern scanning ophthalmic endolaser probe system capable of firing multiple laser spots sequentially in a pattern on the retina through the use of multiple fibers, an optical switching mechanism and a gradient-index (GRIN) lens is disclosed. The design aims to reduce the treatment time of ophthalmic endophotocoagulation and allows for laser shots to be fired at various angles without any moving parts inside the probe. Sequential firing allows precise power level for each treatment spot and low overall output power from the laser unit.

## Description

### FIELD OF THE INVENTION

The present invention consists of an ophthalmic endolaser probe system which aims to reduce endophotocoagulation treatment times by the use of pattern scanning on the retina obtained through an optical switching mechanism, multiple fibers and a gradient index lens.

### BACKGROUND

Fiber optic endolaser probes are commonly used to transmit laser energy for medical applications. Retinal endophotocoagulation involves the introduction of a fiber optic endolaser probe inside the eyeball to apply laser shots to the retina. The proximal end of the probe is attached to an ophthalmic photocoagulation laser usually generating a laser beam in either the 532nm, 577nm or 810 nm wavelength with treatment power levels in the 100-500mW range, and shot durations in the 50-500 msec range, while the distal end is introduced into the intraocular space during a surgical procedure in order to transmit laser energy to the retina.

Retinal endophotocoagulation is a common procedure generally carried after a pars plana vitrectomy for treating a variety of ocular diseases including posterior retinal breaks, stop retinal bleeding, coagulate retinal neovascularization or apply scatter retinal photocoagulation in certain diabetic eyes (Charles S., "Endophotocoagulation", Retina, 1981;1 (2):117-20.). The technique involves shooting laser burns one by one in a grid pattern on the retina and has been routinely used since the early 1980's using endolaser probes containing a single fiber carrying the laser beam (e.g. U.S. Patent 4,537,193 to Tanner). Endolaser probes with illumination capabilities were introduced a decade later, with a single fiber carrying the laser beam, and one or more fibers carrying light from the illumination source to illuminate the treatment area (e.g. U.S. Patent 5,323,766 to Uram).

Since an endophotocoagulation typically requires hundreds of laser shots onto the retina and the treatment is usually carried under general anesthesia, the investigation of new techniques to reduce the treatment time is of primary importance.

One such technique involves firing multiple spots simultaneously from a single laser source by splitting them onto several optical fibers ending on a gradient index (GRIN) lens (e.g. U.S. Patent 8,951,244B2 to Smith). Such splitting techniques introduce a need for a higher power laser and challenges for equal distribution of the laser power among the individual spots.

Retinal photocoagulation without the use of invasive endolaser probes is also common outside the operating room for the treatment of a variety of retinal diseases, including diabetic retinopathy. Such treatments are non-invasive and are carried out without anesthesia, with the patient generally sitting at a slit lamp. Nevertheless, the number of laser shots required is again in the hundreds and reducing treatment time is of importance. Techniques for treating the retina with multiple spots scanned in patterns have been successfully developed for non-invasive photocoagulation. These techniques involve the use of scanners and laser shots at the eye through the cornea without the use of an endolaser probe (e.g. U.S. Pattent 7,766,903B2 to Blumenkranz et al). Tissue-sparing lower power sub-threshold applications using similar setups but with short duration laser pulses are also becoming common practice (e.g. U.S. Patent 7,771,417B2 to Telfair et al).

There is a need for an ophthalmic endolaser probe delivery system that can rapidly transmit the laser energy onto the retina in a pattern of spots, with each spot delivering the same energy; with the said endolaser probe delivery system being connected to commercially available ophthalmic photocoagulation lasers operating within the usual power level range.

### SUMMARY

- The primary object of the invention is to reduce the ophthalmic endophotocoagulation treatment time
- A second object is the placement of spots on the retina in an accurate predictable pattern for improved medical outcome
- A third object is the provision of such an apparatus which is readily usable with existing laser equipment
- A fourth object is the provision to use such an apparatus attached to existing laser systems for the purpose of firing a series of spots in rapid sequence to form a treatment pattern on the retina
- A fifth object is the provision to use such an apparatus attached to existing laser systems for the purpose of firing a single spot to a predetermined position on the retina

### BRIEF DESCRIPTION OF THE DRAWINGS

FIG. 1 is a schematic description of the preferred embodiment of the invention
FIG. 2 is a schematic description of one embodiment of the invention with an angled tip
FIG. 3A shows the principle of operation of the Gradient-Index (GRIN) lens used in the invention (prior art)
FIG. 3B shows the optical fibers and GRIN lens
FIG. 4 shows the practical application in ophthalmology of the preferred embodiment of the invention
FIG. 5 is a schematic of one embodiment of the invention with the option to fire a single laser shot at a selectable angle to the axis of the fibers without the use of moving parts inside the probe

### DETAILED DESCRIPTION

An ophthalmic endolaser probe is an instrument whose proximal end attaches to an ophthalmic photocoagulating laser via a connector and its distal end is inserted inside the eyeball for the purpose of transferring laser energy to treat ophthalmic tissue. The most common application of the endolaser is retinal photocoagulation, i.e. the burning of retinal tissue with laser light. Endolasers are generally used after a vitrectomy procure and typically fire hundreds of laser shots onto the retina. Each shot results in a coagulated spot.

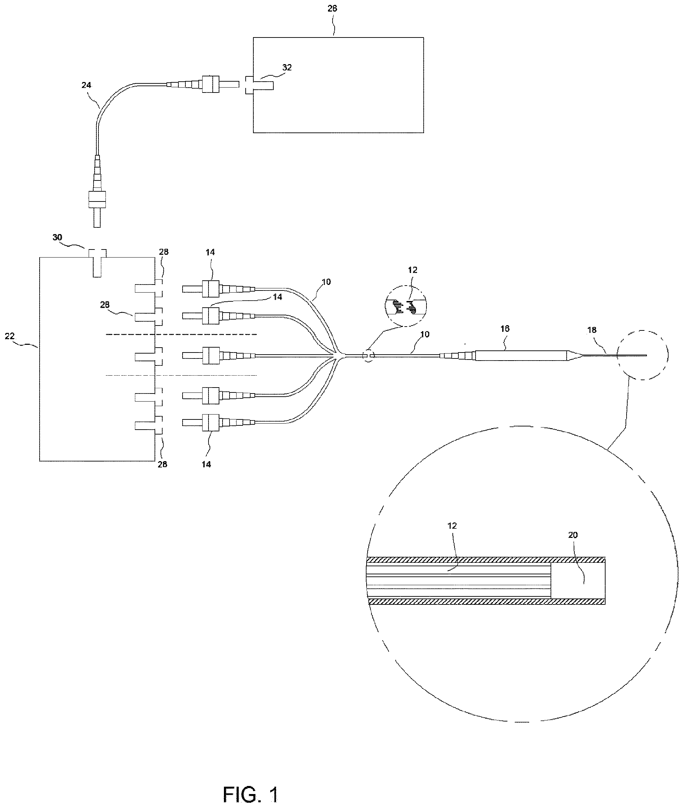

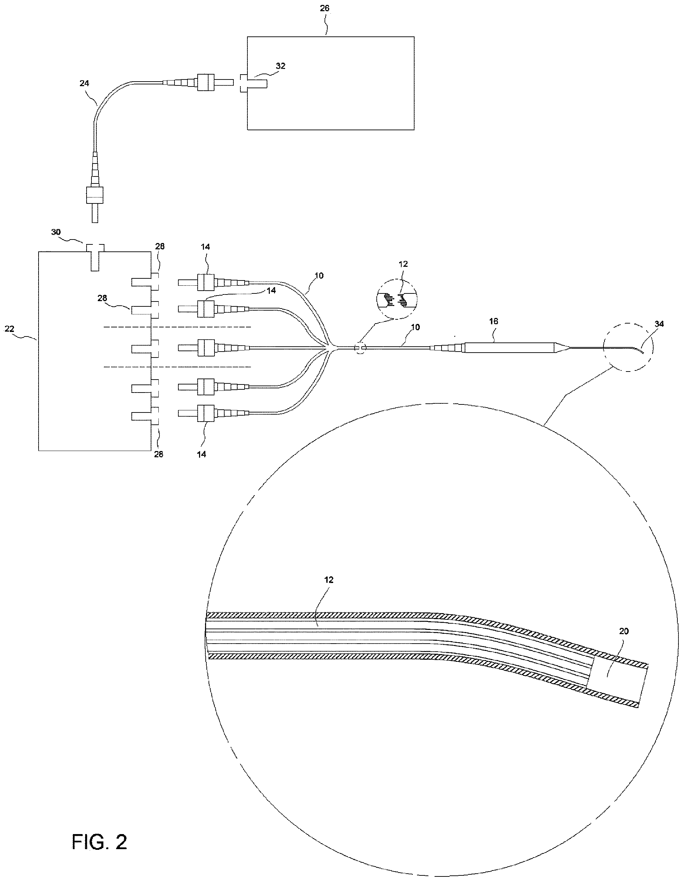

Prior art endolaser probes have a similar external look as the invention in FIG.1 and FIG.2 but lack some critical components unique to the invention. Prior art endolaser probes have a single connector 14, a single optical fiber 12, a protective tubing 10 to protect the fiber, a handpiece 16 for the surgeon to hold the probe and a tip 18 to be inserted inside the eyeball, with some models having a curved or angled tip 34. Some models have multiple fibers 12, with one fiber to carry the laser beam and the others to carry an illumination component.

An optical switch 22 (prior art) is a commercially available device with one input socket 30 and several output sockets 28. Laser energy entering the switch via the input socket 30 is diverted to one of the output sockets 28. Electronic control of the device allows switching from one output socket to another.

The apparatus in the present invention has the same structure as prior art commercially available endolaser probes, but contains multiple connectors 14, multiple fibers 12 and a Gradient -Index (also known as Graded-Index) GRIN lens 20 at the tip, with all fibers 12 attached to an optical switching mechanism 22 through several connectors 14. The optical switching mechanism 22 is itself connected to the output 32 of the laser unit 26 via a fiber optic cable 24. Laser power from the laser unit 26 is sequentially distributed to the output sockets 28 and to the probe through the connectors 14 and the optical fibers 12. The optical switching mechanism 22 allows electronic control of parameters such as the selection of output socket 28 and the duration of the laser shot at each socket.

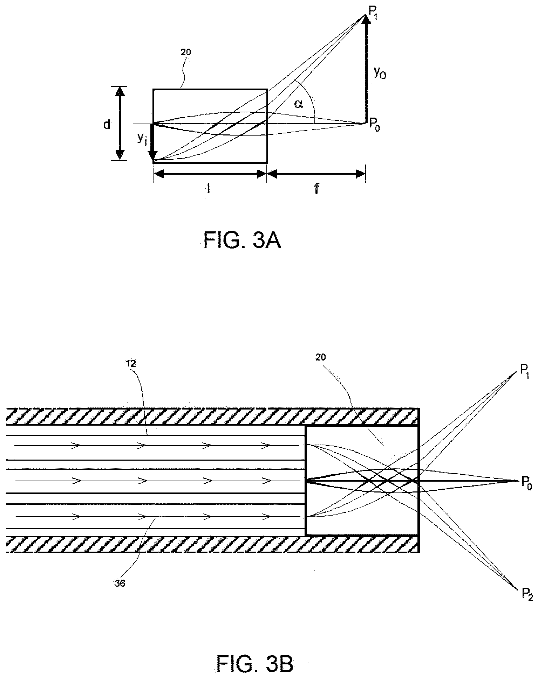

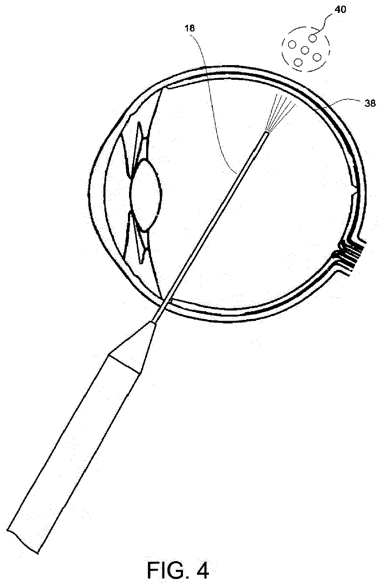

The novelty of the invention is its capability to shoot several laser spots sequentially on the retina 38 in a pattern 40 as shown in FIG.4 through the GRIN lens as described in FIG.3A and FIG.3B. Such a rapid sequence of spots offers the possibility to significantly reduce the treatment time. Compared to simultaneous multiple shots, the sequential pattern approach allows accurate control of the laser power in each individual optical fiber 12 and the use of lower total output power from the laser unit 26.

Synchronizing the optical switching mechanism 22 with the shot duration and interval settings of the laser unit 26 will allow the surgeon to shoot a treatment pattern 40, followed by one or more sequences of aiming beam visual pattern on the retina 38 to determine the next treatment location. This will be followed by another treatment sequence, with the process to be repeated until completion of the treatment.

The operation of a GRIN lens 20 (prior art) is described in FIG.3A. A laser beam entering the GRIN lens 20 at a distance yi from the center initially expands inside the lens, then re-focuses at point P1 with an overall diversion at an angle α from the central axis. When the angle α is such that the distance yo from point P1 to the point P0 on the axis of the lens is greater than the entry distance yi, the laser beam coming from the fiber 12 becomes diverted. FIG.3B shows laser beams 36 entering the GRIN lens 20 from different entry points reaching different output points. The use of multiple fibers 12, each delivering a laser beam to the same GRIN lens 20 will result in the same number of output points at varying angles, hence forming an output pattern 40.

The preferred embodiment of the invention is represented in FIG.1. The apparatus includes several connectors 14 for attachment to an ophthalmic photocoagulation laser 26 via an optical switching mechanism 22, several individual fibers 12 each attached to one connector 14, protective tubings 10 to protect the fibers from external damage, a handpiece 16 to be held by the surgeon and a straight tip 18 containing all the fibers 12 and the GRIN lens 20. The use of this embodiment will result in a sequential coagulation pattern 40 on the retina 38 with as many spots as individual fibers 12 and all laser shots being fired in sequence.

A second embodiment with identical features except for a curved tip 34 is shown in FIG.2. Such an embodiment will allow multiple spots to be fired sequentially at sections inside the eyeball that are difficult to reach with a straight tip.

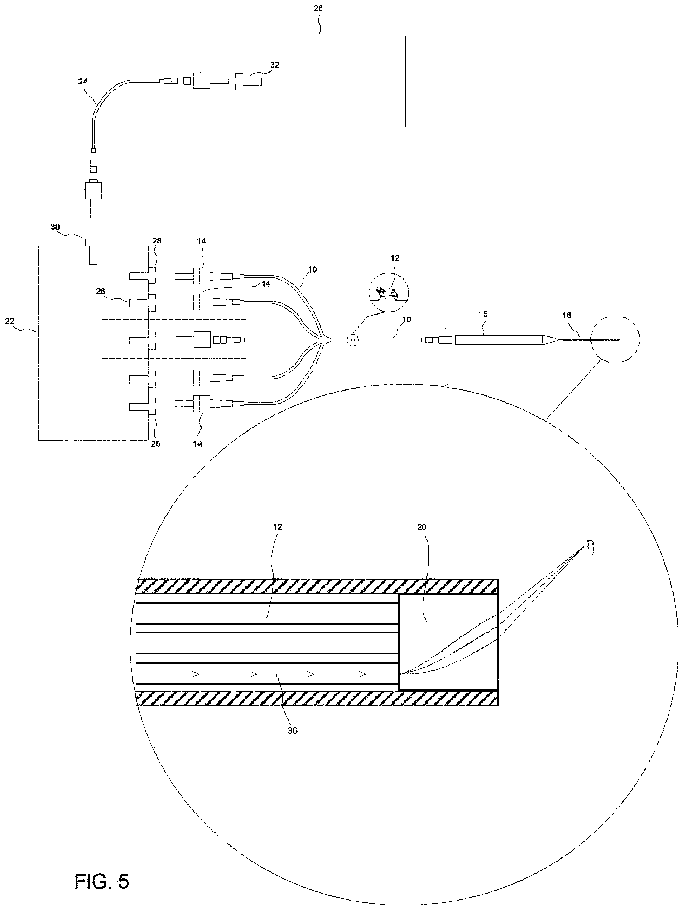

A third embodiment is represented in FIG.5 where the apparatus could be used to shoot single spots straight or at different angles inside the eye without the use of any moving parts inside the probe, by having the optical switching mechanism 22 electronically select a single output socket 28.

### REFERENCES

### PUBLICATIONS

Charles S., "Endophotocoagulation", Retina, 1981;1(2):117-20.

### PATENTS

U.S. Patent 4,537,193 Tanner, "Laser Endocoagulator Apparatus", Aug.27, 1985
U.S. Patent 5,323,766 Uram, "Illuminating Endo-photocoagulation Probe", June 28, 1994
U.S. Patent 8,951,244B2 Smith, "Multi-spot Laser Probe", Feb. 10, 2015
U.S. Pattent 7,766,903B2 Blumenkranz et al, "Patterned Laser Treatment of the Retina", Aug.3, 2010
U.S. Patent 7,771,417B2 Telfair et al, "Laser System with Short Pulse Characteristics and its Methods of Use", Aug. 10, 2010

## Claims

1. A pattern scanning ophthalmic endolaser probe system capable of shooting several laser spots on the retina sequentially in a pattern comprising :
- an optical switching mechanism connected to the ophthalmic photocoagulation laser
- an endolaser probe structure with multiple fibers, each with its own connector
- tubings to protect and hold the fibers
- a handpiece for the surgeon to hold
- a tip for insertion inside the eye, containing the multiple fibers and GRIN lens
- a gradient-index (GRIN) lens located at the output end of the tip to divert the multiple spots in a sequential pattern on the retina

2. The system of claim 1 with an angled probe tip

3. The systems of claim 1 and claim 2 with individually controllable outputs, allowing the firing of a single laser spot on the retina at a selectable angle from the axis of the probe tip without the use of any moving parts inside the probe, the angle being determined by the selection of the output socket of the optical switching mechanism
